(19) **Europäisches Patentamt / European Patent Office / Office européen des brevets**

(11) **EP 4 644 365 A1**

(12) **EUROPEAN PATENT APPLICATION**
published in accordance with Art. 153(4) EPC

(43) Date of publication:
05.11.2025 Bulletin 2025/45

(21) Application number: 23911986.0

(22) Date of filing: 22.12.2023

(51) International Patent Classification (IPC):
*C07C 253/34* (2006.01)    *C07C 253/28* (2006.01)
*C07C 255/51* (2006.01)

(52) Cooperative Patent Classification (CPC):
(C-Sets available)
**C07C 253/28; C07C 253/34**    (Cont.)

(86) International application number:
**PCT/JP2023/046183**

(87) International publication number:
**WO 2024/143206 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: 27.12.2022 JP 2022210507

(71) Applicant: MITSUBISHI GAS CHEMICAL COMPANY, INC.
Chiyoda-ku
Tokyo 100-8324 (JP)

(72) Inventors:
• SUGITA, Takuya
Niigata-shi, Niigata 950-3121 (JP)
• YAMAICHI, Aoto
Niigata-shi, Niigata 950-3121 (JP)
• MISAKI, Satoshi
Niigata-shi, Niigata 950-3121 (JP)
• MUNEYASU, Kuniaki
Niigata-shi, Niigata 950-3121 (JP)

(74) Representative: **Müller-Boré & Partner**
Patentanwälte PartG mbB
Friedenheimer Brücke 21
80639 München (DE)

(54) **METHOD FOR PRODUCING PHTHALONITRILE AND METHOD FOR PURIFYING PHTHALONITRILE**

(57)    A method for producing a phthalonitrile, the method comprising: a production reaction step in which ammonia, oxygen, and xylene are allowed to react in the presence of a catalyst to obtain a reaction product gas containing at least a phthalonitrile and urea; a collection step in which the reaction product gas is brought into contact with an organic solvent to obtain a collection solution; a distillation step in which the collection solution is distilled to separate the phthalonitrile and the organic solvent containing the urea; a water addition step in which water is added to the organic solvent containing the urea; and an aqueous phase separation step in which an aqueous phase containing the urea is separated from the organic solvent.

Fig. 1

**(Cont. next page)**

(52) Cooperative Patent Classification (CPC): (Cont.)

C-Sets
**C07C 253/28, C07C 255/51;**
**C07C 253/34, C07C 255/51**

## Description

### Technical Field

The present invention relates to a method for producing a phthalonitrile and a method for purifying a phthalonitrile.

### Background Art

A method of allowing a carbocyclic compound or heterocyclic compound having an organic substituent, ammonia, and an oxygen-containing gas to react in the presence of a catalyst is called ammoxidation. In the ammoxidation reaction, a vapor phase fluidized catalytic reaction is generally employed. By the ammoxidation reaction, nitrile compounds such as phthalonitriles are produced. Phthalonitriles are useful as production starting materials for synthetic resins, agrochemicals, and others, and as intermediate starting materials for amines, isocyanates, and others. Various methods for producing phthalonitriles by the ammoxidation reaction have been disclosed.

Patent Literature 1 discloses a method for producing isophthalonitrile, the method including: collecting, with an organic solvent, isophthalonitrile in a reaction product gas resulting from the ammoxidation reaction; then the first distillation step of separating a high boiling point impurity; and the second distillation step of volatilizing and separating the organic solvent, to thereby take out isophthalonitrile.

Non-Patent Literature 1 discloses a method including: collecting, with an organic solvent, isophthalonitrile in a reaction product gas; then feeding the collection solution to a solvent recovery column to recover crude isophthalonitrile from the column bottom while removing the solvent from the column top; and then feeding the crude isophthalonitrile to a purification column to recover purified isophthalonitrile from the column top.

### Citation List

### Patent Literature

Patent Literature 1: Japanese Patent Laid-Open No. 2002-097177

### Non-Patent Literature

Non-Patent Literature 1: "Process Handbook" edited and authored by The Japan Petroleum Institute, published by The Japan Petroleum Institute, 1976, MGC-Badger Isophthalonitril Process

### Summary of Invention

### Technical Problem

The organic solvent with which a phthalonitrile produced in the ammoxidation reaction is collected is repeatedly used by separating the phthalonitrile from the organic solvent after the phthalonitrile has been collected. The organic solvent separated from the phthalonitrile contains urea, a byproduct in the ammoxidation reaction, which is accumulated through repeated use of the organic solvent. Since urea has low solubility in the organic solvent, urea tends to be deposited in the solvent tank, solvent discharge pump, various piping, and other places, and the deposited urea needs to be removed in order to avoid blockages in the piping and pump. Accordingly, there has been a need to establish a production method that can stably obtain a phthalonitrile in a high yield over a long period of time by suppressing the deposition of urea.

Meanwhile, neither Patent Literature 1 nor Non-Patent Literature 1 describes a solution to the problem of urea accumulation or deposition in the production process of a phthalonitrile.

An object to be achieved by the present invention is to provide a method for stably producing a phthalonitrile in a high yield over a long period of time, while suppressing the deposition of urea.

### Solution to Problem

As a result of diligent investigations, the present inventors have found that a predetermined production method can suppress deposition of urea and can achieve the above object, thereby completing the present invention.

Specifically, the present invention is as follows.

[1] A method for producing a phthalonitrile, the method comprising:

a production reaction step in which ammonia, oxygen, and xylene are allowed to react in the presence of a catalyst to obtain a reaction product gas containing at least a phthalonitrile and urea;

a collection step in which the reaction product gas is brought into contact with an organic solvent to obtain a collection solution;

a distillation step in which the collection solution is distilled to separate the phthalonitrile and the organic solvent containing the urea;

a water addition step in which water is added to the organic solvent containing the urea; and

an aqueous phase separation step in which an aqueous phase containing the urea is separated from the organic solvent.

[2] The method for producing a phthalonitrile according to [1], wherein, in the water addition step, water is added to the organic solvent in an amount of 10 or more times an entire mass of the urea contained in the organic solvent.

[3] The method for producing a phthalonitrile according to [1] or [2], wherein, in the water addition step, a temperature of the organic solvent is 0°C or higher and lower than 100°C.

[4] The method for producing a phthalonitrile according to any of [1] to [3], wherein the organic solvent is one or more selected from the group consisting of an alkylbenzene, a heterocyclic compound, an aromatic nitrile compound, and a heterocyclic nitrile compound.

[5] The method for producing a phthalonitrile according to any of [1] to [4],

wherein a step series from the production reaction step to the aqueous phase separation step are carried out in succession, and

the organic solvent from which the aqueous phase containing the urea has been separated is used as the organic solvent in the collection step.

[6] The method for producing a phthalonitrile according to any of [1] to [5],

wherein, in the production reaction step, the reaction product gas further contains tolunitrile, and

the tolunitrile is used as the organic solvent in the collection step.

[7] The method for producing a phthalonitrile according to [6], wherein the tolunitrile is m-tolunitrile, p-tolunitrile, or a mixture thereof.

[8] The method for producing a phthalonitrile according to any of [1] to [7], wherein the phthalonitrile is isophthalonitrile, terephthalonitrile, or a mixture thereof.

[9] The method for producing a phthalonitrile according to any of [1] to [8], wherein the distillation step comprises a high boiling separation step in which the collection solution is distilled to separate a high boiling point impurity from the collection solution to obtain a high boiling separation solution.

[10] The method for producing a phthalonitrile according to [9], wherein the high boiling separation step comprises a combustion treatment step in which a column bottom liquid obtained from a column bottom is subjected to a combustion treatment while still in a liquid state, wherein the column bottom liquid has a content of the phthalonitrile of 90% by mass or less and contains the high boiling point impurity.

[11] The method for producing a phthalonitrile according to [9] or [10], wherein the high boiling point impurity is 3-cyanobenzamide, 4-cyanobenzamide, or a mixture thereof.

[12] The method for producing a phthalonitrile according to any of [1] to [11],

wherein a step series from the production reaction step to the aqueous phase separation step are carried out in succession, and

ammonia obtained by thermally decomposing the urea contained in the aqueous phase separated from the organic solvent is used in the production reaction step.

[13] A method for purifying a phthalonitrile, the method comprising:

a collection step in which a reaction product gas containing at least a phthalonitrile and urea is brought into contact with an organic solvent to obtain a collection solution;

a high boiling separation step in which the collection solution is distilled to separate a high boiling point impurity from the collection solution to obtain a high boiling separation solution;

a distillation step in which the high boiling separation solution is distilled to separate the phthalonitrile and the organic solvent containing the urea;

a water addition step in which water is added to the organic solvent containing the urea; and

an aqueous phase separation step in which an aqueous phase containing the urea is separated from the organic solvent.

**Advantageous Effect of Invention**

According to the present invention, there can be provided a method in which a phthalonitrile is stably produced in a high yield over a long period of time by suppressing the deposition of urea.

**Brief Description of Drawing**

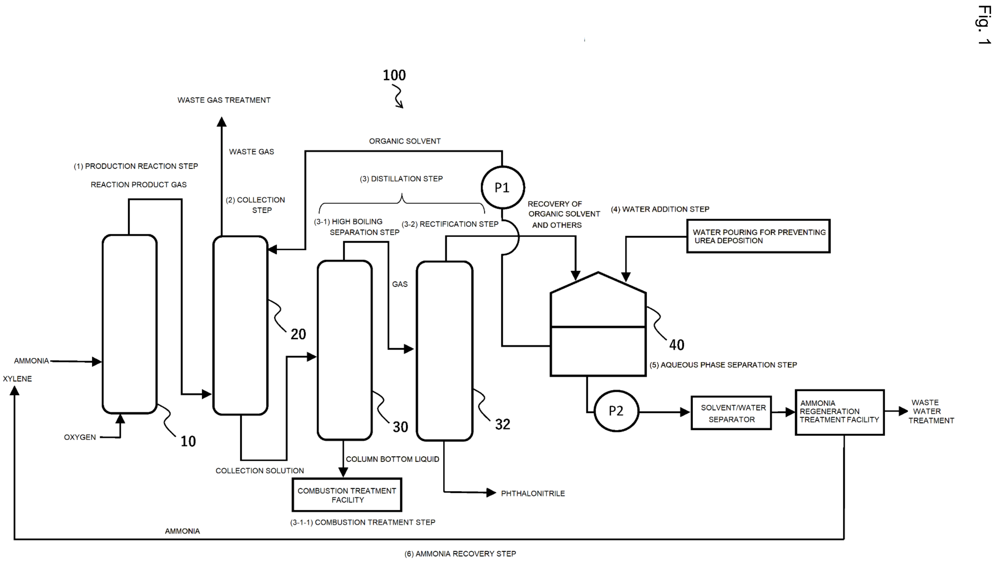

[Figure 1] Figure 1 is a flow diagram illustrating an example of a method for producing a phthalonitrile of the present embodiment.

**Description of Embodiments**

Hereinafter, embodiments of the present invention will be described in detail. Note that the present invention is not limited to the following embodiments, but can be implemented with various modifications within the scope of its gist.

<Method for producing phthalonitrile>

A method for producing a phthalonitrile of the present embodiment includes: (1) a production reaction step in which ammonia, oxygen, and xylene are allowed to react in the presence of a catalyst to obtain a reaction product gas containing at least a phthalonitrile and urea; (2) a collection step in which the reaction product gas is brought into contact with an organic solvent to obtain a collection solution; (3) a distillation step in which the collection solution is distilled to separate the phthalonitrile and the organic solvent containing the urea; (4) a water addition step in which water is added to the organic solvent containing the urea; and (5) an aqueous phase separation step in which an aqueous phase containing the urea is separated from the organic solvent.

(1) Production reaction step

In the production reaction step, an ammoxidation reaction is carried out, in which ammonia, oxygen, and xylene are allowed to react in the presence of a catalyst.
In the production reaction step, the ammoxidation reaction is carried out in a reactor. In the present embodiment, the reaction vessel in which the ammoxidation reaction is carried out is sometimes referred to as "ammoxidation reactor". Since the heat of reaction is large in the ammoxidation reaction, it is preferable to employ a vapor phase fluidized bed reaction in order to obtain a uniform temperature distribution in the ammoxidation reactor. Various types of fluidized bed reactors can be used as the ammoxidation reactor.
The ammonia to be used can be of industrial grade. The molar amount of ammonia to be used is preferably 2 to 20 times the amount of xylene, and more preferably 6 to 15 times the amount of xylene. An excessively small amount to be used may result in a decreased yield of a phthalonitrile. An excessively large amount to be used may decrease the space time yield. Ammonia can be mixed with xylene and fed to the ammoxidation reactor. Under the condition of avoiding the flammability limit, the mixed gas of ammonia and xylene can be further mixed with a portion of an oxygen-containing gas and fed.

The oxygen to be used in the ammoxidation reaction can generally be fed, to the reaction system, in the form of an oxygen-containing gas in which oxygen and another gas coexist. For example, air can be used as the oxygen-containing gas, and oxygen can be fed to the reaction system by feeding the air to the ammoxidation reactor. As the oxygen-containing gas, a gas in which air has been further enriched with oxygen may be used. Also, in the oxygen-containing gas, not only oxygen, but also a diluent such as nitrogen or carbon dioxide gas can be used in combination. The molar amount of oxygen to be used is preferably 3 or more times the amount of xylene, and more preferably 4 to 100 times the amount of xylene. An excessively small amount to be used may result in a decreased yield of a phthalonitrile. An excessively large amount to be used may result in a smaller space time yield.

Examples of the xylene include o-xylene, m-xylene, and p-xylene, but it is preferably m-xylene. In the case where ammoxidation is carried out using air, the concentration of xylene in the starting material gas (gas containing at least ammonia and xylene) fed to the ammoxidation reactor is preferably 0.2 to 10% by volume, and more preferably 0.5 to 5% by volume. An excessively high concentration of xylene may result in a decreased yield of a phthalonitrile. An excessively low xylene concentration may result in a smaller space time yield.

The ammoxidation reaction of ammonia, oxygen, and xylene is carried out in the presence of a catalyst. As the catalyst to be used in the ammoxidation reaction, known catalysts can be used, such as the catalyst containing V-Cr-B-Mo oxides as described in Japanese Patent Laid-Open No. 11-209332 and the catalyst containing Fe-Sb-V oxides as described in Japanese Patent Laid-Open No. 9-71561.

The reaction temperature of ammoxidation is preferably 300 to 500°C, and more preferably 330 to 470°C. An excessively low reaction temperature may result in a lower conversion rate. An excessively high reaction temperature may increase byproducts such as carbon dioxide gas and hydrogen cyanide, and decrease the yield of a phthalonitrile.

The reaction pressure may be any of normal pressure, increased pressure, and reduced pressure, but is preferably in the range of normal pressure (atmospheric pressure, normally 0.1013 MPa in absolute pressure) to 0.3 Mpa, and more preferably in the range of 0.2 to 0.3 Mpa.

The contact time between the gas containing ammonia, oxygen, and xylene and the catalyst depends on conditions such as the molar ratios of charged ammonia and oxygen-containing gas to xylene and the reaction temperature, but is normally in the range of 0.3 to 30 seconds.

In the (1) production reaction step, a reaction product gas containing at least a phthalonitrile and urea is obtained by the ammoxidation reaction. The reaction product gas is recovered from the ammoxidation reactor and subjected to the subsequent (2) collection step.

Examples of the phthalonitrile include isophthalonitrile, terephthalonitrile, and a mixture thereof.

The reaction product gas may further contain a low boiling point impurity such as a byproduct in the ammoxidation reaction. The low boiling point impurity refers to an impurity whose boiling point is lower than that of the phthalonitrile, preferably isophthalonitrile. Examples of the low boiling point impurity include tolunitrile. Examples of the tolunitrile include m-tolunitrile, p-tolunitrile, and a mixture thereof.

In the case where tolunitrile is produced as a byproduct in the production method of the present embodiment, the step series from the (1) production reaction step to the (5) aqueous phase separation step can be carried out in succession, and the tolunitrile produced as a byproduct in the (1) production reaction step can be recovered and used as the organic solvent in the (2) collection step.

The reaction product gas may further contain a high boiling point impurity such as a byproduct in the ammoxidation reaction. The high boiling point impurity refers to an impurity whose boiling point is higher than that of the phthalonitrile, preferably isophthalonitrile. Examples of the high boiling point impurity include cyanobenzamide. Examples of the cyanobenzamide include 3-cyanobenzamide, 4-cyanobenzamide, and a mixture thereof. Other than the cyanobenzamide, m-toluamide, 3-cyanobenzoic acid, and others may also be contained.

(2) Collection step

In the collection step, the reaction product gas is brought into contact with an organic solvent to obtain a collection solution.

In the present embodiment, the column in which the collection step is carried out is sometimes referred to as "collection column". The reaction product gas recovered from the ammoxidation reactor is introduced into the collection column and comes into contact with an organic solvent. When the reaction product gas comes into contact with an organic solvent, at least the phthalonitrile and urea in the reaction product gas are dissolved in the organic solvent. In the present embodiment, the "collection solution" means an organic solvent after contact with the reaction product gas, the organic solvent containing at least the phthalonitrile and urea. At the top of the collection column, an absorption part including a tray or packed layer may be installed. In this case, the organic solvent is fed from the top of the collection column.

As the organic solvent, one with a lower boiling point than that of the phthalonitrile is used. The organic solvent is preferably at least one or more selected from the group consisting of an alkylbenzene, a heterocyclic compound, an aromatic nitrile compound, and a heterocyclic nitrile compound. An organic solvent is more preferable such that the solubility of the phthalonitrile in the organic solvent is high and also such that the organic solvent is inert to the phthalonitrile. Also, in view of separating an aqueous phase from the organic solvent in the (5) aqueous phase separation step, an organic solvent with a low solubility in water is more preferable.

Specific examples of the organic solvent include m-xylene, pseudocumene, mesitylene, ethylbenzene, methylpyridine, benzonitrile, m-tolunitrile, p-tolunitrile, and cyanopyridine, and m-tolunitrile is more preferable. One of these organic solvents can be used alone, or some of them can be used in combination.

As mentioned above, the low boiling point impurity resulting from the (1) reaction production step may be used as the organic solvent.

The temperature of the collection column is set such that the organic solvent before coming into contact with the reaction product gas and the collection solution that have piled up at the column bottom (hereinafter, these are sometimes collectively referred to as "liquid phase at the column bottom") are at a temperature of the boiling point or lower. In detail, the compositional features of the liquid phase at the column bottom in the (2) collection step depend on the amount of the reaction product gas from the ammoxidation reactor and the amount of the organic solvent to be fed to the collection column, and the temperature at the column bottom of the collection column is set such that the liquid phase at the column bottom with the compositional features in each occasion is at a temperature of the boiling point or lower.

The pressure in the collection column may be any of normal pressure, increased pressure, and reduced pressure, but is normally set in the range of normal pressure to the ammoxidation reaction pressure.

Not only in the (1) production reaction step, but also in the (2) collection step, a high boiling point impurity other than cyanobenzamide, such as m-toluamide and 3-cyanobenzoic acid mentioned above, may be generated in the collection column.

In the (2) collection step, the reaction product gas is blown into the liquid phase at the column bottom of the collection column. This allows the phthalonitrile in the reaction product gas to be dissolved in the organic solvent and collected, along with the high boiling point impurity such as cyanobenzamide and, in some cases, the low boiling point impurity. On the other hand, unreacted ammonia, a hydrophilic byproduct such as hydrogen cyanide, carbon dioxide, water, carbon monoxide, nitrogen, oxygen, and others are not collected in the organic solvent and are separated as a waste gas. The waste gas is discharged from the top of the collection column. The collection solution is recovered from the column bottom part of the collection column and is subjected to the subsequent (3) distillation step.

(3) Distillation step

In the distillation step, the collection solution is distilled to separate the phthalonitrile and the organic solvent containing the urea.

In the present embodiment, "separation" in the distillation step is not limited to a manner (i) in which the phthalonitrile and the organic solvent containing the urea are separated directly by distillation of the collection solution, but may be in a manner (ii) in which the collection solution is distilled to separate a mixture containing at least the phthalonitrile, urea, and organic solvent from the high boiling point impurity (high boiling separation step described later), and then the mixture containing the phthalonitrile, urea, and organic solvent is rectified to separate the phthalonitrile from the organic solvent containing the urea (rectification step described later). Also, the manner (ii) may be a manner in which the phthalonitrile and the organic solvent containing the urea are separated through multiple steps after distillation of the collection solution, such as a manner in which a portion of the phthalonitrile is separated together with the high boiling point impurity from the mixture containing the organic solvent and others. Hereinafter, the distillation step will be described on an exemplary mode in which it includes a high boiling separation step and a rectification step.

(3-1) High boiling separation step

In the present embodiment, the distillation step may include a high boiling separation step in which the collection solution is distilled to separate a high boiling point impurity from the collection solution to obtain a high boiling separation solution.

In the present embodiment, the column in which the high boiling separation step is carried out is sometimes referred to as "high boiling separation column". The high boiling separation column can be configured to distill the collection solution, and to recover a gaseous mixture containing the phthalonitrile, urea, organic solvent, and in some cases low boiling point impurity from the column top and recover a column bottom liquid containing the high boiling point impurity from the column bottom. In the high boiling separation step, distillation apparatuses such as a packed column, a tray column, and a flash drum can be used. Also, the high boiling separation step can be performed under reduced pressure in a batch or continuous manner.

In the present embodiment, the column bottom liquid of the high boiling separation column may further contain the phthalonitrile, as mentioned above. In this case, in the high boiling separation step, specifically, the content of the phthalonitrile is preferably 90% by mass or less, more preferably 60% by mass or less, still more preferably 40% by mass or less, particularly preferably 20% by mass or less, further particularly preferably 10% by mass or less, and most preferably less than 10% by mass. The lower limit of the content of the phthalonitrile is not particularly limited, but it is preferably 5% by mass or more.

As the method for measuring the content, gas chromatography is preferable. The content is preferably measured after completion of the high boiling separation step. The column bottom liquid is recovered while still in a liquid state.

The column bottom liquid of the high boiling separation column can contain the high boiling point impurity such as cyanobenzamide and the phthalonitrile. The phthalonitrile is unstable to heat in the coexistence of impurities such as cyanobenzamide, ammoxidation catalyst, and metal, and is prone to alteration such as amidation or polymerization. Alteration is more likely to occur and to proceed to a greater degree, as the phthalonitrile is exposure to high temperature for a longer time or to a higher temperature. Therefore, in order to carry out the high boiling separation step quickly at a temperature as low as possible, it is preferable to control the conditions in the high boiling separation column, specifically, the column bottom temperature, the residence time of the column bottom liquid at the column bottom, and the distillation pressure. By doing so, alteration of the phthalonitrile can be less likely to progress. This can suppress the rising of the melting point of the components contained in the column bottom liquid, and therefore suppress the rising of the viscosity of the column bottom liquid. As a result, it is expected that a loss of the phthalonitrile due to alteration of the phthalonitrile can be suppressed. It is also expected that solidification blockage due to alteration of the column bottom liquid can be prevented.

Specifically, the distillation pressure of the column bottom liquid is preferably 12 kPa or less, more preferably less than 12 kPa, and particularly preferably less than 8 kPa. The lower limit of the distillation pressure is not particularly limited, but it is preferably 5 kPa or more.

The temperature of the column bottom liquid is preferably 200 to 230°C, more preferably 210 to 230°C, and particularly preferably 220 to 230°C. Note that, if the temperature of the column bottom liquid is lower than 200°C, it is below the melting point of the high boiling point impurity such as cyanobenzamide, which may cause the column bottom liquid to be solidified.

The liquid residence time of the column bottom liquid is preferably shorter than 96 hours, and more preferably 72 hours or shorter. The lower limit of the liquid residence time is not particularly limited and may be 0 hours or longer, but it is preferably 12 hours or longer, more preferably 24 hours or longer, and particularly preferably 48 hours or longer.

It is preferable that the distillation pressure of the column bottom liquid should be 5 to 12 kPa, the temperature thereof should be 200 to 230°C, and the liquid residence time should be 12 hours or longer and within 72 hours. It is more preferable that the distillation pressure of the column bottom liquid should be 5 kPa or more and less than 12 kPa, the temperature thereof should be 210 to 230°C, and the liquid residence time should be 12 hours or longer and within 72 hours. It is particularly preferable that the distillation pressure of the column bottom liquid should be 5 kPa or more and less than 8 kPa, the temperature thereof should be 220 to 230°C, and the liquid residence time should be 12 hours or longer and within 72 hours.

The liquid residence time can be calculated according to the following expression:

$$X = B/A$$

wherein X (hr) represents the liquid residence time, A ($m^3$/hr) represents the amount of the column bottom liquid discharged, and B ($m^3$) represents the amount of liquid held at the column bottom of the high boiling separation column. In the case where water vapor is generated from the heat generated by a combustion treatment and is fed to the high boiling separation column, B represents the amount of liquid held at the column bottom of the high boiling separation column, piping for column bottom liquid circulation extending from the column bottom, and a reboiler.

The column bottom liquid recovered from the column bottom may be subjected to a combustion treatment step described later. On the other hand, the gas recovered from the column top (mixture containing the phthalonitrile, urea, and organic solvent) is introduced into a rectification column where the (3-2) rectification step is carried out.

(3-1-1) Combustion treatment step

In the present embodiment, the high boiling separation step may further include the combustion treatment step in which the column bottom liquid obtained from the column bottom is subjected to a combustion treatment while still in a liquid state, wherein the column bottom liquid has a content of the phthalonitrile of 90% by mass or less and contains the high boiling point impurity.

In the combustion treatment step of the present embodiment, the column bottom liquid is transferred to an incinerator for combustion while still in a liquid state, without solidifying or gasifying the column bottom liquid as much as possible. The column bottom liquid is maintained in a temperature range above the melting point and below the boiling point during transfer to the incinerator. As the means to transfer the column bottom liquid while maintaining this temperature range, known means may be utilized, such as heating the piping through which the column bottom liquid is passed with a heater or the like. It is preferable to pass the column bottom liquid through the inner side of double piping and a fluid for temperature maintenance, such as steam, through the outer side.

For the transfer of the column bottom liquid and the feed thereof to the incinerator, it is preferable to use a pump. As the incinerator, known incinerators can be used. For example, horizontal cylindrical furnaces, vertical cylindrical furnaces, and the like can be used. The column bottom liquid may be blown in a liquid state, with a nozzle into the incinerator for combustion.

The heat generated by the combustion treatment can be utilized for the generation of water vapor. For example, a water drum, a pipe through which water is passed (hereinafter, referred to as water pipe), and a steam drum are installed in the incinerator (boiler) in which the column bottom liquid is combusted, and the water in the water pipe is heated by the heat generated by the combustion, thereby generating water vapor. The generated water vapor is sent out of the incinerator through piping for water vapor that is kept warm.

The generated water vapor can be utilized for various applications. For example, by placing piping for water vapor between the combustion treatment facility and the piping for column bottom liquid, and by sending water vapor into the piping for column bottom liquid, the temperature of the column bottom liquid during transfer can be maintained. As the piping for column bottom liquid, double piping is used. It is preferable to make the piping for column bottom liquid have a structure that circulates the column bottom liquid while discharging a portion thereof, to provide a reboiler (also called heat exchanger) in the circulation part of the piping for column bottom liquid, and to feed water vapor to the reboiler.

Also, by placing the piping for water vapor between the combustion treatment facility and the piping for collection solution extending from the collection column, the high boiling separation column, or the rectification column, water vapor can be utilized as a heat source in the (2) collection step or the (3) distillation step.

Furthermore, by placing the piping for water vapor up to the piping for phthalonitrile extending from the rectifying column, water vapor can be utilized as a heat source to prevent consolidation of the phthalonitrile as it falls below the melting point. As the piping for phthalonitrile, double piping is used. It is preferable to make the piping for phthalonitrile have a structure that circulates the phthalonitrile while discharging a portion thereof, to provide a reboiler (also called heat exchanger) in the circulation part of the piping for phthalonitrile, and to feed water vapor to the reboiler.

(3-2) Rectification step

In the production method of the present embodiment, the distillation step can be configured to include the high boiling separation step and the rectification step, as mentioned above. The rectification step is a step in which the mixture containing the phthalonitrile, urea, and organic solvent, which has been at least separated from the high boiling point impurity in the high boiling separation step, is distilled (rectified) to separate the phthalonitrile from the organic solvent containing the urea.

The gas (mixture) obtained from the column top in the high boiling separation step contains not only the target phthalonitrile but also other components such as the urea, organic solvent, and low boiling point impurity. Therefore, in the (3-2) rectification step, the phthalonitrile is separated from these other components. In the present embodiment, the column in which the distillation step is carried out is sometimes referred to as "rectification column". Specifically, distillation is carried out in the rectification column to remove the organic solvent containing the urea from the column top, and to recover the target purified phthalonitrile in a liquid state from the column bottom.

In subjecting the gas obtained in the high boiling separation step to the rectification step, the mixture containing the phthalonitrile, urea, and organic solvent may be introduced into the rectification column in a gaseous state or may be condensed and introduced into the rectification column in a liquid state. However, in view of reducing the amount of energy used in the distillation step, it is preferable to introduce the mixture into the rectification step while still in a gaseous state.

The operation pressure of the rectification column is preferably reduced pressure. Specifically, for the operation pressure of the rectification column, a high vacuum condition is selected such that the phthalonitrile is not deposited in the column. For example, the pressure in the rectification column in the case where m-tolunitrile is used as the organic solvent is preferably in the range of 5 to 10 kPa. No scrubber may be needed between the condensation part and the vacuum exhaust part in the case where the following conditions are satisfied: the phthalonitrile is in contact with a sufficient amount of organic solvent in the rectification column; and the main component of the condensation part is the solvent at a low temperature while there is no vapor pressure of the phthalonitrile in the condensation part, so that the phthalonitrile does not scatter into the vacuum exhaust system.

In the rectification column, the mixture containing the phthalonitrile, urea, and organic solvent is distilled, and the organic solvent containing the urea is recovered from the column top and the target phthalonitrile is recovered in a liquid state from the column bottom.

The organic solvent containing the urea recovered from the column top of the rectification column is subjected to the subsequent (4) water addition step.

(4) Water addition step

In the water addition step, water is added to the organic solvent containing the urea. The water addition step is a step provided to suppress urea from being deposited in the facility (in particular, facilities where the organic solvent after recovery flows (solvent tank, near the inlet side of the solvent discharge pump, heat exchanger, and others)) by removing the urea contained in the organic solvent when the organic solvent recovered from the (3) distillation step is reused in the (2) collection step. In the production method of the present embodiment, the urea in the organic solvent is distributed to the water added in the present step, and the urea can thus be removed from the organic solvent. In order to promote distribution of the urea in the organic solvent to the added water, the organic solvent to which water has been added may be stirred. The stirring method is not particularly limited, but examples thereof include a method involving mechanical stirring with a stirring blade or a mixer, and a method involving water flow stirring by adding water to cause a water flow. In the case where stirring is carried out, it is preferable to allow the mixture to stand still in order to separate the organic solvent phase from the aqueous phase containing the urea. In the case where the organic solvent to which water has been added is allowed to reside in the solvent tank, the residence time may be used as the time to allow the mixture to stand still.

The organic solvent containing the urea recovered from the column top of the rectification column or the like in the (3) distillation step is transferred to a solvent tank or the like and subjected to the water addition step. The organic solvent recovered from the column top of the rectification column or the like may be cooled by a heat exchanger or the like and introduced into a solvent tank in a liquid state.

The mass of the water to be added to the organic solvent is preferably 10 or more times, more preferably 50 or more times, and particularly preferably 100 or more times the entire mass of the urea contained in the organic solvent. When the amount of the water to be added to the organic solvent is in the above range, the urea in the organic solvent can be more efficiently distributed to the water, and deposition of the urea can be further suppressed. In the case where the mass of the water to be added to the organic solvent is 10 or more times the entire mass of the urea contained in the organic solvent, deposition of the urea in the solvent tank, solvent discharge pump, various piping, and other places can be suppressed. The upper limit of the mass of the water to be added to the organic solvent is not particularly limited, but it is preferably less than 500 times the entire mass of the urea contained in the organic solvent. When the mass of the water to be added to the organic solvent is less than 500 times the entire mass of the urea contained in the organic solvent, it is possible to avoid increased load on various apparatuses and increased waste water volume.

The entire mass of the urea contained in the organic solvent can be determined by calculation from the urea concentration in the organic solvent. As the method for measuring the urea concentration in the organic solvent, measurement by high performance liquid chromatography is preferable. The urea concentration in the organic solvent is preferably measured in the distillation step and in the aqueous phase separation step. In the case where the entire mass of the urea contained in the organic solvent is determined from the urea concentration by calculation, the calculation can be performed assuming that the entire volume of the organic solvent containing the urea is the same as the volume of the solvent tank.

The temperature of the organic solvent at the time of water pouring is preferably 0°C or higher and lower than 100°C, more preferably 10°C or higher and lower than 80°C, and particularly preferably 20°C or higher and lower than 60°C. In the case where the temperature of the organic solvent is 0°C or higher and lower than 100°C, the urea in the organic solvent can be more efficiently distributed to the water, and deposition of the urea can be further suppressed.

As for the temperature of the organic solvent, it is preferable to measure the temperature of the organic solvent in the solvent tank.

The temperature of the organic solvent can be regulated by a heat exchanger such as chiller. In the case where the temperature is regulated by a heat exchanger, the position where the heat exchanger is provided is not particularly limited, but in view of regulating the temperature of the organic solvent more efficiently, it is preferable to provide it between the rectification column and the solvent tank.

In the present embodiment, the position where water is added to the organic solvent containing urea is referred to as water pouring site. The water pouring site can be provided near the location where urea is likely to be deposited, and for example, can be provided at the solvent tank, near the inlet side of the solvent discharge pump, or, in the case where cooling is performed by a heat exchanger or the like, near the inlet side of the heat exchanger, for example. A plurality of water pouring sites may be provided, and in that case the mass of the water to be added is controlled in consideration of the addition of water from the plurality of water pouring sites. In Figure 1 described later, an apparatus provided with a water pouring site at a solvent tank is exemplified, but the position of the water pouring site is not limited to the mode of Figure 1.

In the (4) water addition step, the urea contained in the organic solvent is distributed to the added water, resulting in an aqueous phase containing the urea. The aqueous phase containing the urea is subjected to the subsequent (5) aqueous phase separation step.

(5) Aqueous phase separation step

In the aqueous phase separation step, the aqueous phase containing the urea is separated from the organic solvent.

In the solvent tank, water pouring separates the organic solvent phase from the aqueous phase containing the urea. In the case where the aqueous phase containing the urea is the lower layer, the aqueous phase containing the urea is discharged from the solvent tank through the lower layer discharge pump (shown in Figure 2), allowing the aqueous phase containing the urea to be separated from the organic solvent. The organic solvent that has been separated from the aqueous phase containing the urea is recovered from the upper part of the solvent tank by the solvent discharge pump.

In the present embodiment, it is preferable to carry out the (1) production reaction step to the (3) aqueous phase separation step in succession, and to use again the organic solvent from which the aqueous phase containing the urea has been separated as the organic solvent in the (2) collection step. The (4) water addition step and the (5) aqueous phase separation step make it possible to remove urea from the organic solvent, and even if the organic solvent is used repeatedly in the collection step, urea is not deposited in the solvent tank, solvent discharge pump, various piping, and other places. This prevents blockages in the piping and pump, and allows stable production of the phthalonitrile in a high yield over a long period of time.

The aqueous phase discharged from the solvent tank is subjected to a waste water treatment after separating the organic solvent and urea therefrom. Also, the organic solvent separated from the aqueous phase may be returned to the solvent tank for reuse.

(6) Ammonia recovery step

In the production method of the present embodiment, it is preferable that the step series from the (1) production reaction step to the (5) aqueous phase separation step should be carried out in succession, and that in the (5) aqueous phase separation step, ammonia obtained by thermally decomposing the urea contained in the aqueous phase separated from the organic solvent should be used again in the (1) production reaction step. It is preferable that, by heating the aqueous phase containing the urea separated from the organic solvent, the urea should be thermally decomposed to obtain ammonia, and that the ammonia should be then reused in the (1) production reaction step. The temperature at which the urea is thermally decomposed is not particularly limited, but it is preferably 125°C or higher and 165°C or lower, and more preferably 125°C or higher and 150°C or lower. By reusing ammonia in the ammoxidation reaction, the starting material intensity can be improved and the phthalonitrile can be produced in a high yield.

<Method for purifying phthalonitrile>

The present invention also provides a method for purifying a phthalonitrile.

The method for purifying a phthalonitrile of the present embodiment includes:

a collection step in which a reaction product gas containing at least a phthalonitrile and urea is brought into contact with an organic solvent to obtain a collection solution;

a high boiling separation step in which the collection solution is distilled to separate a high boiling point impurity from the collection solution to obtain a high boiling separation solution;

a distillation step in which the high boiling separation solution is distilled to separate the phthalonitrile and the organic solvent containing the urea;

a water addition step in which water is added to the organic solvent containing the urea; and

an aqueous phase separation step in which an aqueous phase containing the urea is separated from the organic solvent.

The details of the collection step, high boiling separation step, distillation step, water addition step, and aqueous phase separation step are as described in the description pertaining to the production method of the present embodiment.

<Method for producing phthalonitrile of the present embodiment>

Hereinafter, an example of a method for producing a phthalonitrile of the present embodiment will be described with reference to Figure 1. Figure 1 is a flow diagram illustrating an example of the method for producing a phthalonitrile of the present embodiment. However, the production method of the present embodiment is not limited to the mode shown in Figure 1. In Figure 1, a facility 100 for achieving the production method of the present embodiment is configured to include an ammoxidation reactor 10, a collection column 20, a high boiling separation column 30, a rectification column 32, and a solvent tank 40.

In Figure 1, the (1) production reaction step is carried out in the ammoxidation reactor 10. As shown in Figure 1, ammonia, oxygen, and xylene are fed into the ammoxidation reactor 10, and when they come into contact with a catalyst installed in the ammoxidation reactor 10, which is not shown in the figure, the ammoxidation reaction is initiated. As shown in Figure 1, ammonia and xylene can be mixed and fed to the ammoxidation reactor 10. The ammoxidation reaction of the starting materials fed into the ammoxidation reactor 10 produces a reaction product gas containing at least a phthalonitrile and urea. The reaction product gas is discharged from the column top part of the ammoxidation reactor 10 and fed to the collection column 20.

The (2) collection step is carried out in the collection column 20. In the collection column 20, there is provided an absorption part including a tray or packed layer, which is not shown in the figure, and an organic solvent is fed from the top. The reaction product gas fed to the collection column 20 comes into contact with the organic solvent fed from the top of the collection column 20, and the phthalonitrile and urea are dissolved in the organic solvent. The organic solvent in which the phthalonitrile and others are dissolved then piles up at the column bottom and is recovered as a collection solution from the column bottom part of the collection column 20. In the mode shown in Figure 1, in addition to the phthalonitrile, urea, and organic solvent, a high boiling point impurity is contained in the collection solution. The collection solution recovered from the collection column 20 is fed to the high boiling separation column 30 of the (3) distillation step.

In Figure 1, the (3) distillation step includes the (3-1) high boiling separation step and the (3-2) rectification step. The collection solution is distilled in the high boiling separation column 30 and the phthalonitrile is separated from the organic solvent containing the urea in the rectification column 32.

The (3-1) high boiling separation step is carried out in the high boiling separation column 30. The collection solution fed to the high boiling separation column 30 is distilled in the high boiling separation column 30, and a gaseous mixture containing at least the phthalonitrile, urea, and organic solvent is discharged from the column top. The gas discharged from the high boiling separation column 30 is fed to the rectification column 32. On the other hand, at the column bottom part of the high boiling separation column 30, a column bottom liquid containing the high boiling point impurity separated from the collection solution piles up. The column bottom liquid is recovered from the column bottom part of the high boiling separation column 30. As shown in Figure 1, the recovered column bottom liquid is subjected to a combustion treatment while still in a liquid state in a combustion treatment facility for (3-1-1) combustion treatment step.

The (3-2) rectification step is carried out in the rectification column 32. The gaseous mixture containing the phthalonitrile, urea, and organic solvent fed to the rectification column 32 is again distilled (rectified) in the rectification column 32 to separate the phthalonitrile from the organic solvent containing the urea. The organic solvent containing the urea separated from the phthalonitrile is discharged from the column top part of the rectification column 32 while still in a gaseous state and is fed to the solvent tank 40 of the (4) water addition step. On the other hand, the purified phthalonitrile separated from the organic solvent and others is recovered from the column bottom part of the rectification column 32. Also, the urea concentration in the organic solvent is measured in the (3) distillation step and/or the (5) aqueous phase separation step by, for example, high performance liquid chromatography. After adding water to the organic solvent containing the urea, the urea is extracted into the aqueous phase and the urea concentration is measured by high performance liquid chromatography. As an example of the measurement method by high performance liquid chromatography, the following conditions can be used: mobile phase: water, flow rate: 0.5 ml/min, column temperature: 40°C, detector: UV 195 nm, and column: Shodex DE-613 (particle diameter: 6 $\mu$m, inner diameter: 6 mm, length: 150 mm).

In Figure 1, the (4) water addition step and the (5) aqueous phase separation step are carried out in the solvent tank 40. However, in the production method of the present embodiment, the water pouring site can be provided in piping or the like before feeding to the solvent tank 40 or after feeding to the solvent tank. In the case where the water pouring site is provided only in a place other than the solvent tank 40, the (4) water addition step is carried out in a facility other than the solvent tank 40, for example, in piping equipped with a water pouring site before feeding to the solvent tank 40.

The organic solvent containing the urea recovered from the rectification column 32 is discharged into the solvent tank 40. The solvent tank 40 is equipped with a water pouring function for preventing urea deposition, and a water pouring site is provided so that a predetermined amount of water can be fed into the tank. When water is added to the organic solvent in the solvent tank 40, the organic solvent is phase-separated into an organic solvent phase not containing urea and an aqueous phase containing urea. The organic solvent phase in the solvent tank 40 is sent to the collection column 20 of the (2) collection step by driving of a pump P1 and is reused. On the other hand, the aqueous phase containing urea is discharged from the bottom side of the solvent tank 40 by driving of a pump P2 in the (5) aqueous phase separation step. The aqueous phase discharged from the solvent tank 40 is subjected to a waste water treatment via an ammonia regeneration treatment facility after the organic solvent is removed by a solvent/water separator as shown in Figure 1. Meanwhile, in Figure 1, ammonia can be recovered in the (6) ammonia recovery step, from the urea contained in the aqueous phase discharged from the solvent tank 40. The aqueous phase containing urea discharged from the solvent tank 40 is heated in an ammonia regeneration treatment facility (for example, ammonia regeneration tower) after the organic solvent is removed by a solvent/water separator. The urea contained in the aqueous phase is thermally decomposed by heating, producing ammonia. The ammonia produced in the ammonia regeneration treatment facility is sent to the ammoxidation reactor 10 of the (1) production reaction step to be reused. As mentioned above, the aqueous phase after separation may be reused again as the water for preventing urea deposition after removing urea and others.

According to the production method of the present embodiment, when the recovered organic solvent is reused in the collection step, urea has been removed in advance, which effectively suppresses deposition of urea in the solvent tank 40, pump P1, piping between the solvent tank 40 and the collection column 20, and other places where urea is otherwise easy to be deposited. This makes it possible to stably produce a phthalonitrile in a high yield over a long period of time.

Examples

Hereinafter, the present embodiment will be described further specifically by way of Examples and Comparative Examples, but the present embodiment is not limited to these Examples only.

(Experimental Example 1)

Water was added to m-tolunitrile containing urea in a glass container to examine the deposition of urea.

The concentration of urea contained in m-tolunitrile was set to 500 ppm, 1000 ppm, or 5000 ppm, and a certain amount of water was added to the m-tolunitrile. Subsequently, the amount of urea deposition (%) was determined by the method for measuring the amount of urea deposition, and the degree of urea deposition was determined according to the criteria in Table 1. The temperature of m-tolunitrile was 35°C. The results are shown in Table 2.

[Method for measuring the amount of urea deposition]

A solvent was placed in a glass container, and a predetermined amount of water and urea were added to prepare a mixed solution. Thereafter, the mixed solution was stirred well and allowed to stand still. After allowing the mixed solution to stand still for about 1 hour, it was filtered (filter: Omnipore PTFE 10.0 $\mu$m, washing solvent: diethyl ether). After drying the filter paper and the glass container at 60°C for 3 hours, the weight of the filter paper and the glass container was measured with a precision balance. The proportion, to the amount of urea added, of the total increased weight obtained by subtracted the tare of the filter paper and glass container from the found value of the weight was defined as the amount of urea deposition (%). The amount of urea deposition (%) was evaluated on a 5-point scale in Table 1 to determine the effect of preventing deposition of urea.

[Table 1]

| Point | Amount of urea deposition (%) |
|---|---|
| V: Very effective in preventing blockages | 0.0-14.9 |
| IV: Effective in preventing blockages | 15.0-29.0 |
| III: Moderately effective in preventing blockages | 30.0-39.9 |
| II: Slightly effective in preventing blockages | 40.0-99.9 |
| I: Not effective in preventing blockages | 100 |

In the case where water was not added to m-tolunitrile (Comparative Example 1), urea was deposited in 100% and the effect of preventing blockages was not obtained. On the other hand, in the case where water was added in an amount of 5 to 20 times the entire mass of the urea contained in m-tolunitrile (Examples 1 to 9), the deposition of urea was suppressed and the effect of preventing blockages was obtained. It was found that the addition of water and particularly the addition of water in an amount of 10 or more times the entire mass of the urea contained in m-tolunitrile further suppressed the deposition of urea and further provide the effect of preventing blockages.

[Table 2]

|  | m-Tolunitrile | Water | Urea | Amount of water added | Urea concentration in solvent | Amount of urea deposition | Effect of preventing deposition |
|---|---|---|---|---|---|---|---|
|  | 9 | 9 | 9 | Amount relative to the entire mass of urea | ppm | % | - |
| Comparative Example 1 | 100 | 0.0 | 0.1 | 0 times | 1000 | 100 | I: Not effective in preventing blockages |
| Example 1 | 100 | 0.5 | 0.1 | 5 times | 1000 | 32.3 | III: Moderately effective in preventing blockages |
| Example 2 | 100 | 1.0 | 0.1 | 10 times | 1000 | 22.4 | IV: Effective in preventing blockages |
| Example 3 | 100 | 2.0 | 0.1 | 20 times | 1000 | 13.8 | V: Very effective in preventing blockages |
| Example 4 | 100 | 0.3 | 0.05 | 6 times | 500 | 47.7 | II: Slightly effective in preventing blockages |
| Example 5 | 100 | 0.5 | 0.05 | 10 times | 500 | 39.9 | Iii* Moderately effective in preventing blockages |
| Example 6 | 100 | 1.0 | 0.05 | 20 times | 500 | 31.0 | III: Moderately effective in preventing blockages |
| Example 7 | 100 | 3.0 | 0.5 | 6 times | 5000 | 6.5 | V: Very effective in preventing blockages |
| Example 8 | 100 | 5.0 | 0.5 | 10 times | 5000 | 4.3 | V::Very effective in preventing blockages |
| Example 9 | 100 | 10.0 | 0.5 | 20 times | 5000 | 2.5 | V: Very effective in preventing blockages |

(Experimental Example 2)

Water was added to m-xylene, pseudocumene, or mesitylene containing urea to examine the deposition of urea.

The concentration of urea contained in m-xylene, pseudocumene, or mesitylene was adjusted to 1000 ppm, and a certain amount of water was added to those organic solvents. Subsequently, after stirring the mixture well and allowing it to stand still, the degree of urea deposition was determined according to the same criteria as in Experimental Example 1 shown in Table 1. The temperature of the organic solvents was 35°C. The results are shown in Tables 3 to 5.

In the case where water was not added to m-xylene, pseudocumene, or mesitylene (Comparative Examples 2 to 4), urea was deposited in 100% and the effect of preventing blockages was not obtained. On the other hand, in the case where water was added in an amount of 5 to 20 times the entire mass of the urea contained in m-xylene, pseudocumene, or mesitylene (Examples 10 to 18), the deposition of urea was suppressed and the effect of preventing blockages was obtained. It was also found that the addition of water in an amount of 10 or more times the entire mass of the urea contained in m-xylene, pseudocumene, or mesitylene further suppressed the deposition of urea and further provide the effect of preventing blockages.

[Table 3]

| | m-Xylene | Water | Urea | Amount of water added | Urea concentration in solvent | Amount of urea deposition | Effect of preventing deposition |
|---|---|---|---|---|---|---|---|
| | 9 | 9 | 9 | Amount relative to the entire mass of urea | ppm | % | - |
| Comparative Example 2 | 100 | 0.0 | 0.1 | 0 times | 1000 | 100 | Not effective |
| Example 10 | 100 | 0.5 | 0.1 | 5 times | 1000 | 31.2 | III: Moderately effective in preventing blockages |
| Example 11 | 100 | 1.0 | 0.1 | 10 times | 1000 | 18.0 | IV: Effective in preventing blockages |
| Example 12 | 100 | 2.0 | 0.1 | 20 times | 1000 | 9.1 | V: Very effective in preventing blockages |

[Table 4]

| | Pseudocumene | Water | Urea | Amount of water added | Urea concentration in solvent | Amount of urea deposition | Effect of preventing deposition |
|---|---|---|---|---|---|---|---|
| | 9 | 9 | 9 | Amount relative to the entire mass of urea | ppm | % | - |
| Comparative Example 3 | 100 | 0.0 | 0.1 | 0 times | 1000 | 100 | Not effective |
| Example 13 | 100 | 0.5 | 0.1 | 5 times | 1000 | 46.0 | II: Slightly effective in preventing blockages |
| Example 14 | 100 | 1.0 | 0.1 | 10 times | 1000 | 24.0 | IV: Effective in preventing blockages |
| Example 15 | 100 | 2.0 | 0.1 | 20 times | 1000 | 13.2 | V: Very effective in preventing blockages |

[Table 5]

| | Mesitylene | Water | Urea | Amount of water added | Urea concentration in solvent | Amount of urea deposition | Effect of preventing deposition |
|---|---|---|---|---|---|---|---|
| | 9 | 9 | 9 | Amount relative to the entire mass of urea | ppm | % | - |
| Comparative Example 4 | 100 | 0.0 | 0.1 | 0 times | 1000 | 100 | Not effective |
| Example 16 | 100 | 0.5 | 0.1 | 5 times | 1000 | 30.7 | III: Moderately effective in preventing blockages |
| Example 17 | 100 | 1.0 | 0.1 | 10 times | 1000 | 16.9 | IV: Effective in preventing blockages |
| Example 18 | 100 | 2.0 | 0.1 | 20 times | 1000 | 14.1 | V: Very effective in preventing blockages |

(Example 19)

After going through the production reaction step, the collection step, and the distillation step, water was added in an amount of 50 times that of urea, to m-tolunitrile containing 5 kg of urea in a solvent tank, as the water addition step. The liquid temperature in the solvent tank was 35°C. Thereafter, when the aqueous phase was separated in the aqueous phase separation step, urea was not deposited in the pump, inside the piping, and other places even after 300 days had elapsed.

(Comparative Example 5)

After going through the production reaction step, the collection step, and the distillation step, water was not added to m-tolunitrile containing 5 kg of urea in a solvent tank and the aqueous phase separation step was not carried out either. The liquid temperature in the solvent tank was 35°C. As a result, after 90 days had elapsed, urea was deposited in the pump, inside the piping, and other places, resulting in blockages. In consequence, it became difficult to continue the operation.

**Reference Signs List**

10 ammoxidation reactor, 20 collection column, 30 high boiling separation column, 32 rectification column, 40 solvent tank.

**Claims**

1.    A method for producing a phthalonitrile, the method comprising:

a production reaction step in which ammonia, oxygen, and xylene are allowed to react in the presence of a catalyst to obtain a reaction product gas containing at least a phthalonitrile and urea;
a collection step in which the reaction product gas is brought into contact with an organic solvent to obtain a collection solution;
a distillation step in which the collection solution is distilled to separate the phthalonitrile and the organic solvent containing the urea;
a water addition step in which water is added to the organic solvent containing the urea; and
an aqueous phase separation step in which an aqueous phase containing the urea is separated from the organic solvent.

2. The method for producing a phthalonitrile according to claim 1, wherein, in the water addition step, water is added to the organic solvent in an amount of 10 or more times an entire mass of the urea contained in the organic solvent.

3. The method for producing a phthalonitrile according to claim 1 or 2, wherein, in the water addition step, a temperature of the organic solvent is 0°C or higher and lower than 100°C.

4. The method for producing a phthalonitrile according to any one of claims 1 to 3, wherein the organic solvent is one or more selected from the group consisting of an alkylbenzene, a heterocyclic compound, an aromatic nitrile compound, and a heterocyclic nitrile compound.

5. The method for producing a phthalonitrile according to any one of claims 1 to 4,

   wherein a step series from the production reaction step to the aqueous phase separation step are carried out in succession, and
   the organic solvent from which the aqueous phase containing the urea has been separated is used as the organic solvent in the collection step.

6. The method for producing a phthalonitrile according to any one of claims 1 to 5,

   wherein, in the production reaction step, the reaction product gas further contains tolunitrile, and
   the tolunitrile is used as the organic solvent in the collection step.

7. The method for producing a phthalonitrile according to claim 6, wherein the tolunitrile is m-tolunitrile, p-tolunitrile, or a mixture thereof.

8. The method for producing a phthalonitrile according to any one of claims 1 to 7, wherein the phthalonitrile is isophthalonitrile, terephthalonitrile, or a mixture thereof.

9. The method for producing a phthalonitrile according to any one of claims 1 to 8, wherein the distillation step comprises a high boiling separation step in which the collection solution is distilled to separate a high boiling point impurity from the collection solution to obtain a high boiling separation solution.

10. The method for producing a phthalonitrile according to claim 9, wherein the high boiling separation step comprises a combustion treatment step in which a column bottom liquid obtained from a column bottom is subjected to a combustion treatment while still in a liquid state, wherein the column bottom liquid has a content of the phthalonitrile of 90% by mass or less and contains the high boiling point impurity.

11. The method for producing a phthalonitrile according to claim 9 or 10, wherein the high boiling point impurity is 3-cyanobenzamide, 4-cyanobenzamide, or a mixture thereof.

12. The method for producing a phthalonitrile according to any one of claims 1 to 11,

   wherein the step series from the production reaction step to the aqueous phase separation step are carried out in succession, and
   ammonia obtained by thermally decomposing the urea contained in the aqueous phase separated from the organic solvent is used in the production reaction step.

13. A method for purifying a phthalonitrile, the method comprising:

   a collection step in which a reaction product gas containing at least a phthalonitrile and urea is brought into contact with an organic solvent to obtain a collection solution;
   a high boiling separation step in which the collection solution is distilled to separate a high boiling point impurity from the collection solution to obtain a high boiling separation solution;
   a distillation step in which the high boiling separation solution is distilled to separate the phthalonitrile and the organic solvent containing the urea;
   a water addition step in which water is added to the organic solvent containing the urea; and
   an aqueous phase separation step in which an aqueous phase containing the urea is separated from the organic solvent.

Fig. 1

100

**(1) PRODUCTION REACTION STEP**
AMMONIA
XYLENE
OXYGEN
REACTION PRODUCT GAS
10

**(2) COLLECTION STEP**
WASTE GAS
WASTE GAS TREATMENT
20
COLLECTION SOLUTION

**(3) DISTILLATION STEP**
ORGANIC SOLVENT
**(3-1) HIGH BOILING SEPARATION STEP**
GAS
30
COLUMN BOTTOM LIQUID
**(3-1-1) COMBUSTION TREATMENT STEP**
COMBUSTION TREATMENT FACILITY
**(3-2) RECTIFICATION STEP**
32
PHTHALONITRILE
P1
RECOVERY OF ORGANIC SOLVENT AND OTHERS

**(4) WATER ADDITION STEP**
WATER POURING FOR PREVENTING UREA DEPOSITION
40

**(5) AQUEOUS PHASE SEPARATION STEP**
P2
SOLVENT/WATER SEPARATOR
AMMONIA REGENERATION TREATMENT FACILITY
WASTE WATER TREATMENT

**(6) AMMONIA RECOVERY STEP**
AMMONIA

18

## INTERNATIONAL SEARCH REPORT

| International application No. |
| --- |
| **PCT/JP2023/046183** |

**A.    CLASSIFICATION OF SUBJECT MATTER**

*C07C 253/34*(2006.01)i; *C07C 253/28*(2006.01)i; *C07C 255/51*(2006.01)i
FI:    C07C253/34; C07C255/51; C07C253/28

According to International Patent Classification (IPC) or to both national classification and IPC

**B.    FIELDS SEARCHED**

Minimum documentation searched (classification system followed by classification symbols)

C07C253/34; C07C253/28; C07C255/51

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| A | WO 2021/172499 A1 (MITSUBISHI GAS CHEMICAL COMPANY, INC.) 02 September 2021 (2021-09-02)<br>claims, examples, drawings | 1-13 |
| A | JP 2002-97177 A (MITSUBISHI GAS CHEMICAL COMPANY, INC.) 02 April 2002 (2002-04-02)<br>claims, examples | 1-13 |
| A | WO 2020/203581 A1 (MITSUBISHI GAS CHEMICAL COMPANY, INC.) 08 October 2020 (2020-10-08)<br>claims, examples, drawings | 1-13 |
| A | WO 2015/137267 A1 (MITSUBISHI GAS CHEMICAL COMPANY, INC.) 17 September 2015 (2015-09-17)<br>claims, examples, drawings | 1-13 |

☐ Further documents are listed in the continuation of Box C.    ☑ See patent family annex.

| | |
| --- | --- |
| *    Special categories of cited documents:<br>"A"  document defining the general state of the art which is not considered to be of particular relevance<br>"D"  document cited by the applicant in the international application<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search | Date of mailing of the international search report |
| --- | --- |
| **27 February 2024** | **19 March 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
Information on patent family members

International application No.

**PCT/JP2023/046183**

| Patent document cited in search report | | | Publication date (day/month/year) | Patent family member(s) | | | Publication date (day/month/year) |
|---|---|---|---|---|---|---|---|
| WO | 2021/172499 | A1 | 02 September 2021 | US | 2023/0111802 | A1 | |
| | | | | claims, examples, figures | | | |
| | | | | EP | 4112598 | A1 | |
| | | | | CN | 115175897 | A | |
| | | | | KR | 10-2022-0147578 | A | |
| JP | 2002-97177 | A | 02 April 2002 | US | 2002/0035287 | A1 | |
| | | | | claims, examples | | | |
| | | | | WO | 2003/082810 | A1 | |
| | | | | EP | 1193247 | A2 | |
| | | | | TW | 587070 | B | |
| | | | | KR | 10-2002-0023133 | A | |
| | | | | CN | 1520396 | A | |
| WO | 2020/203581 | A1 | 08 October 2020 | US | 2022/0135515 | A1 | |
| | | | | claims, examples, figures | | | |
| | | | | EP | 3822254 | A1 | |
| | | | | CN | 113557225 | A | |
| | | | | TW | 202043191 | A | |
| WO | 2015/137267 | A1 | 17 September 2015 | US | 2017/0217873 | A1 | |
| | | | | claims, examples, figures | | | |
| | | | | EP | 3118188 | A1 | |
| | | | | KR | 10-2016-0003887 | A | |
| | | | | CN | 106068254 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002097177 A
- JP 11209332 A
- JP 9071561 A

**Non-patent literature cited in the description**

- MGC-Badger Isophthalonitril Process. Process Handbook. The Japan Petroleum Institute, 1976